# FASCICULE DE BREVET EUROPEEN

(11) **EP 0 878 700 B1**
(45) Date de publication et mention de la délivrance du brevet: **30.07.2003**
(21) Numéro de dépôt: 97460018.1
(22) Date de dépôt: 16.05.1997
(51) Int. Cl.: G01N 1/14

(54) **Installation automatique de surveillance de la qualité d'un milieu liquide, notamment aquatique**
Automatisches Überwachungssystem für die Qualität flüssiger, insbesondere aquatischer, Medien
Automatic system for monitoring the quality of a liquid medium, particularly an aquatic medium

(43) Date de publication de la demande: 18.11.1998
(73) Titulaire: IFREMER INSTITUT FRANCAIS DE RECHERCHE POUR L'EXPLOITATION DE LA MER, 92138 Issy-les-Moulineaux Cédex (FR)
(72) Inventeur: Woerther, Patrice, 29280 Plouzane (FR); Leilde, Bernard, 29200 Brest (FR); Jegou, Paul, 29200 Brest (FR)
(74) Mandataire: Bentz, Jean-Paul

(56) Documents cités:
- EP-A- 0 079 283
- WO-A-95/24627
- DE-A- 3 739 772
- FR-A- 2 688 885
- US-A- 4 594 903

## Description

La présente invention concerne une installation automatique de surveillance de la qualité d'un milieu liquide comprenant un système de prélèvement d'échantillon liquide associé à un circuit hydraulique doté de capteurs de mesure et de détection pour procéder à un examen de cet échantillon. Elle concerne plus particulièrement une telle installation destinée à la surveillance de la qualité de l'eau douce ou de l'eau de mer en un lieu déterminé.

La surveillance de la qualité de l'eau, notamment dans les zones côtières, les estuaires, les rivières, les lacs ou autres retenues d'eau tend à se généraliser, en premier lieu dans le cadre du contrôle de la qualité des eaux, de la détection des pollutions, de la modélisation des écosystèmes, mais aussi dans le but de mieux maîtriser certaines opérations, liées à l'aquaculture par exemple, telles que l'irrigation des claires d'affinage d'huîtres.

Dans un souci d'efficacité, on a pensé à mettre au point des installations automatiques. Pour l'essentiel, elles comprennent un système de prélèvement d'échantillon liquide pouvant de préférence opérer sélectivement à différentes profondeurs, associé à un circuit hydraulique pourvu de capteurs de mesure et de détection, dans lequel un flux d'échantillon liquide à partir d'un orifice de prélèvement est assuré au moyen d'une pompe. Les installations sont en principe appelées à fonctionner en continu, ou tout au moins selon des durées relativement longues réparties sur la journée, sous la commande et le contrôle d'un automate. Les données issues des capteurs parviennent à une unité de traitement délivrant des informations utiles sur place ou à distance. Dans le cas d'installations se trouvant au large, le transfert d'informations se fait par voie hertzienne.

C'est plus précisément à ces installations destinées à être basées au large que se rapporte l'invention, du fait que leur fonctionnement dans des conditions correctes donne naissance à une problématique multiple.

Une première contrainte est liée à l'énergie disponible, en ce sens qu'une installation de très faible puissance pourra être avantageusement alimentée de façon autonome au moyen de récupérateurs d'énergie naturelle tels qu'éoliennes ou piles solaires. En contre-partie, il faudra pallier aux inconvénients induits par la limitation de puissance au niveau de la pompe assurant le flux d'échantillon liquide dans le circuit, qui pourra être sujette à des dysfonctionnements ou désamorçages dus notamment à la présence de gaz dans le circuit, amenés par l'échantillon liquide.

Un autre problème réside dans la protection du circuit contre l'encrassement des conduites résultant en grande partie de la prolifération biologique. Cette protection doit être constante pour que le flux d'échantillon liquide dans le circuit soit suffisamment régulier pour ne pas affecter les mesures.

L'encrassement concerne également les capteurs du circuit. Leur précision et/ou leur sensibilité est également diminuée par l'accumulation sur leur tête de bulles gazeuses qui s'y fixent lors du passage de l'échantillon liquide.

L'invention a consisté dans l'apport d'une solution globale satisfaisante aux problèmes énoncés ci-dessus, de manière à obtenir des données fiables à partir d'installations autonomes fonctionnant durablement de façon fiable.

A cet effet, elle consiste en une installation de surveillance de la qualité d'un milieu liquide comprenant, associé à un système de prélèvement d'échantillon dans le milieu liquide, un circuit hydraulique doté de capteurs de mesure et/ou de détection dans lequel passe ledit échantillon, caractérisée en ce que ledit circuit comprend d'amont en aval à partir de l'arrivée d'échantillon en provenance du système de prélèvement :
une colonne montante le long de laquelle sont implantés lesdits capteurs ;
une première pompe au-dessus de la colonne montante assurant la circulation de l'échantillon dans ledit circuit ;
un désaérateur pour piéger les gaz transportés sous forme de bulles par l'échantillon liquide, lequel désaérateur est placé au-dessus de ladite première pompe ;
une conduite de refoulement raccordée à la base dudit désaérateur et pourvue d'une vanne d'arrêt ;
une seconde pompe auto-amorçante montée sur une conduite raccordée au sommet dudit désaérateur, et
un moyen pour soumettre périodiquement les têtes d'au moins certains des capteurs à des jets de liquide sous pression pour en détacher les salissures et bulles gazeuses qui s'y fixent lors du passage de l'échantillon.

La seconde pompe auto-amorçante sert pour l'essentiel au remplissage en liquide dudit circuit, et à l'évacuation régulière des gaz recueillis dans le désaérateur. Etant donné qu'elle est destinée à fonctionner très peu en temps, elle peut être choisie relativement puissante même s'il y a obligation de se tenir à une gestion serrée de l'énergie. En revanche, le fonctionnement de la première pompe au-dessus de ladite colonne montante est permanent ou quasi-permanent. Mais comme sa seule fonction est d'entretenir le flux d'échantillon dans le circuit, sa puissance peut être limitée, jusqu'à moins de dix Watts en pratique.

Dans les applications à la surveillance de l'eau, il convient d'empêcher l'encrassage des conduites et éléments de circuit dû notamment à la prolifération biologique. A cet effet, il est prévu selon une autre caractéristique de l'invention de monter à la base de ladite colonne montante un moyen générateur de chlore ou chlorateur, connu en soi. Dans une forme de réalisation préférée, ledit clorateur est conçu de manière à former un fond de décantation pour ladite colonne montante.

En outre, toujours pour prévenir l'encrassage interne du circuit, il pourra être prévu que dans au moins certaines parties du circuit et du système de prélèvement, les éléments ou parois au contact de l'échantillon liquide soient constitués ou revêtus d'un matériau antifouling (antisallissure) tel que du cupronickel.

Selon une autre caractéristique de l'invention, le désaérateur comporte une enceinte cylindrique verticale communiquant par sa base avec la sortie de ladite première pompe et la conduite de refoulement, et par son sommet avec l'entrée de ladite seconde pompe auto-amorçante. Le désaérateur est pourvu d'un détecteur de niveau minimum de liquide dans l'enceinte pour lequel il convient d'évacuer les gaz recueillis.

Selon une autre caractéristique de l'invention, ledit moyen pour soumettre des têtes de capteur à des jets de liquide sous pression comporte une conduite dotée d'une troisième pompe, connectée en amont de celle-ci à la conduite de refoulement, et desservant en aval une pluralité de conduites secondaires se terminant chacune par un ajutage dirigé sur une tête de capteur.

Selon une autre caractéristique de l'invention, un débitmètre est prévu dans ledit circuit, destiné à régler le flux d'échantillon liquide dans le circuit, et à détecter des anomalies affectant ledit flux, telles que des colmatages.

Selon une autre caractéristique de l'invention, ledit système de prélèvement d'échantillons comporte une pluralité de conduites de prélèvement dont les orifices de prélèvement sont à des profondeurs différentes, lesquelles conduites sont toutes raccordables sélectivement avec l'entrée et la sortie dudit circuit.

Selon une autre caractéristique de l'invention, toute la partie dudit circuit à partir de l'orifice de prélèvement d'échantillon jusqu'au désaérateur est montante.

D'autres caractéristiques et avantages de l'invention apparaîtront à la lecture de la description suivante d'une forme de réalisation donnée à titre d'exemple seulement, et faite en relation avec les dessins joints, dans lesquels :
la Fig. 1 est une vue schématique en élévation du circuit hydraulique d'une installation de surveillance selon l'invention ; et
la Fig. 2 est une vue de dessus en coupe, prise selon la ligne II-II de la Fig. 1.

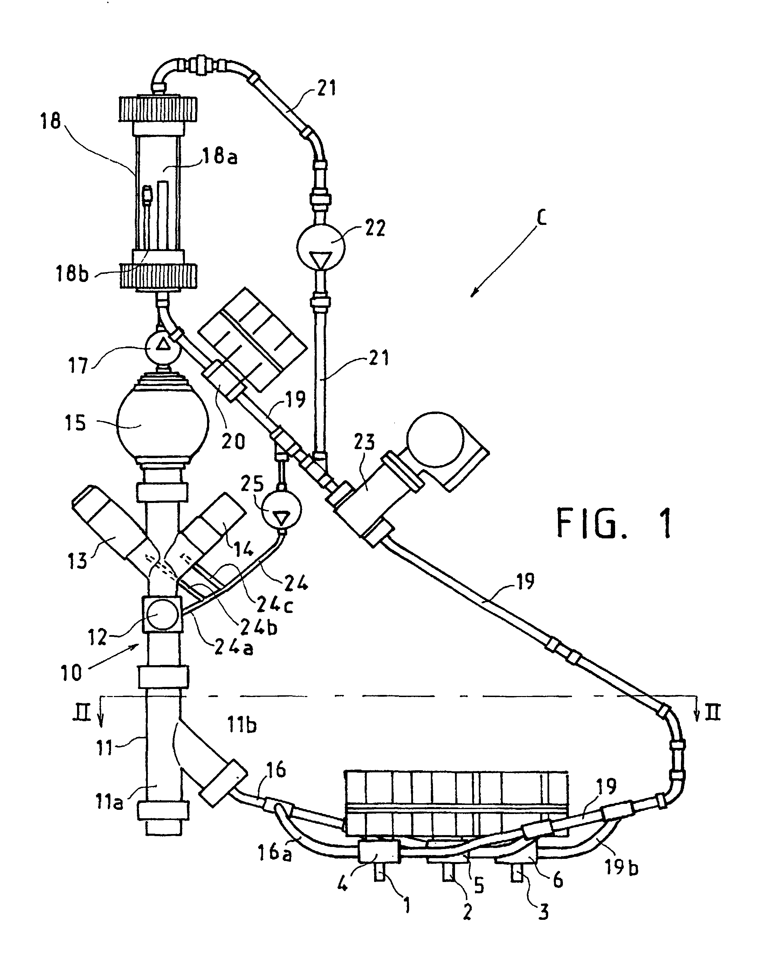

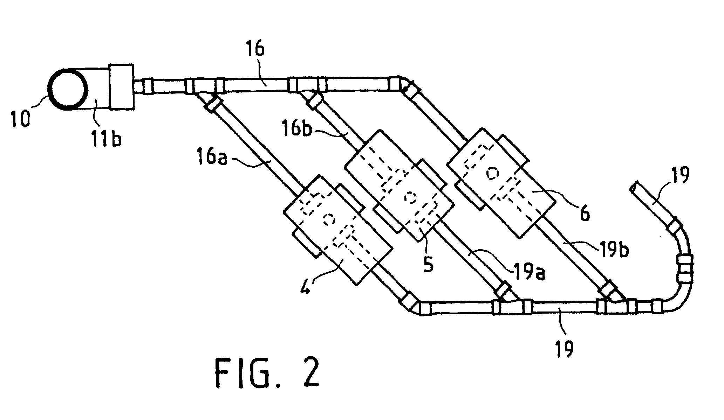

Les Figs. 1 et 2 représentent le circuit hydraulique C doté de capteurs d'une installation de surveillance de la qualité de l'eau, plus spécialement destinée à fonctionner de façon autonome à distance du rivage, par exemple au milieu ou au large d'un estuaire.

En régle générale, l'installation est destinée à être montée sur un caisson flottant, non représenté, ancré sur le fond. Son système de prélèvement d'échantillon, également non représenté, est avantageusement du type décrit dans la demande de brevet français n° 94 02880 aux noms du présent demandeur et de la Société MORS, intitulée "dispositif de prélèvement de liquide tel que de l'eau de mer ou de l'eau douce, en vue d'en mesurer les paramètres physico-chimiques". Ce dispositif de prélèvement comporte une pluralité de conduites débouchant à des profondeurs différentes. Elles sont représentées à la Fig. 1, au nombre de trois, par leurs extrémités supérieures 1, 2 et 3, respectivement raccordées aux premiers orifices de vannes trois voies 4, 5 et 6. En pratique les conduites 1 à 3 sont toutes montantes depuis leur orifice de prélèvement jusqu'à leur extrémité supérieure.

Le circuit C comprend une colonne montante 10 sur la hauteur de laquelle sont implantés de bas en haut un chlorateur 11 et différents capteurs dont des capteurs 12 à 14 réalisant des mesures (pH, conductivité, taux de turbidité, taux d'oxygène dissous, etc.), et des capteurs de détection de la présence ou de traces d'éléments divers, tels qu'un fluorimètre 15 pour la chlorophylle.

Dans la forme représentée à la Fig. 1, le chlorateur 11 comporte une partie tubulaire verticale 11a sur laquelle se raccorde en Y inversé, à une certaine distance au-dessus de sa base, une partie tubulaire oblique 11b communiquant avec une conduite 16 assurant la liaison avec le système de prélèvement pour l'admission d'échantillon liquide. Plus précisément, la conduite 16 est raccordée directement avec le second orifice de la vanne trois voies 6, et par l'intermédiaire de ramifications 16a et 16b, Fig. 2, avec les seconds orifices des vannes trois voies 4 et 5, pour communiquer sélectivement avec l'une quelconque des conduites de prélèvement 1 à 3. On pourra remarquer en observant la Fig. 1 que la portion de circuit à partir des vannes trois voies 5 à 6 comprenant la conduite 16 et ses ramifications 16a, 16b, et la partie tubulaire 11b, est montante vers la colonne montante 10.

D'autre part, la base de la partie tubulaire 11a au-dessous du raccordement avec la partie tubulaire 11b constitue le fond de la colonne montante 10 et peut donc être utilisée avantageusement en tant que décanteur pour recueillir une partie des sédiments transportés par l'échantillon liquide.

La colonne montante 10 est surmontée d'une première pompe 17 assurant la circulation d'échantillon liquide dans le circuit C, à partir de l'orifice de prélèvement. Comme on l'a mentionné précédemment, la pompe 17, par exemple de type centrifuge, peut être de faible puissance.

Un désaérateur 18 occupe le sommet du circuit C, au-dessus de la première pompe 17. Tel que montré, il comprend une enceinte cylindrique verticale 18a de récupération des gaz et un détecteur de niveau minimal d'eau dans l'enceinte 18a. Une conduite de sortie de la première pompe 17 débouche dans la partie inférieure de l'enceinte 18a, en traversant le fond 18b, tandis qu'en repart, en traversant également le fond 18b, une conduite de refoulement 19 du circuit C, sur laquelle est montée une vanne d'arrêt 20. Du sommet de l'enceinte 18a, part une conduite 21 qui rejoint la conduite de refoulement 19 en aval de la vanne d'arrêt 20. Sur la conduite 21, est montée une pompe auto-amorçante 22.

En pratique, la conduite de sortie de la première pompe 17 et la conduite de refoulement 19 traversent le fond 18b du désaérateur, leurs orifices respectifs dans l'enceinte 18a se trouvant à un niveau supérieur à celui du fond 18b, de sorte que le désaérateur 18 peut faire office également de décanteur, comme le chlorateur 11.

Sur la conduite de refoulement 19, en aval du point de raccordement avec la conduite 21, est monté un débitmètre 23 servant à contrôler la circulation de l'eau dans le circuit C. En aval du débitmètre 23, la conduite de refoulement 19 est raccordée directement au troisième orifice de la vanne trois voies 4, et par des ramifications 19a, 19b, Fig.2, aux troisièmes orifices respectifs des vannes trois voies 5 et 6. Eventuellement, il peut être prévu un ou plusieurs piquages sur la conduite de refoulement 19, en aval du débitmètre 23, pour dériver une partie d'échantillon vers des capteurs-analyseurs et/ou des moyens de récupération et de préservation. On notera que la partie du circuit C constituée par la conduite de refoulement 19 et ses ramifications 19a, 19b est continûment descendante depuis le désaérateur 18 jusqu'aux vannes trois voies 4, 5, 6, et donc au-delà jusqu'à l'orifice du prélèvement par lequel l'échantillon d'eau sort du circuit C. En revanche, dans la partie amont du circuit, le flux d'échantillon est continûment montant depuis l'orifice de prélèvement jusqu'au désaérateur 18.

Selon l'invention, il est en outre prévu dans le circuit C un moyen pour évacuer des têtes de détection d'au moins certains des capteurs, ici les capteurs 12 à 14, les salissures et les bulles gazeuses qui ont tendance à s'y fixer lors du fonctionnement. Tel que représenté à la Fig. 1, ledit moyen comprend une conduite 24 dotée d'une pompe 25, raccordée en amont de celle-ci à la conduite de refoulement 19, entre la vanne d'arrêt 20 et le débitmètre 23, et qui alimente en pression des conduites secondaires 24a, 24b, 24c pénétrant dans la colonne montante 10 et se terminant par des ajutages dirigés chacun sur une tête de détection de capteur.

La première étape du fonctionnement de l'installation est la mise en eau du circuit C qui s'effectue au moyen de la pompe auto-amorçante 22, après que la vanne d'arrêt 20 a été fermée, la conduite d'admission d'échantillon 16 mise en communication avec une des conduites de prélèvement 1 à 3, et la conduite de refoulement 19 mise en communication avec une autre des conduites 1 à 3 (les entrée et sortie d'échantillon étant donc à des profondeurs différentes).

Ensuite, la vanne 20 est ouverte à nouveau, la pompe 22 est arrêtée, et la première pompe 17 prend le relais, pour assurer un flux d'échantillon liquide dans le circuit C qui est alors apte à fonctionner. Le nettoyage des têtes des capteurs 12 à 14 est réalisé par simple mise en route momentanée de la pompe 25. Les données en provenance des capteurs 12 à 15 sont dès lors exploitables.

Le flux d'échantillon liquide entretenu par la pompe 17 dans le circuit C est interrompu lorsque l'accumulation de gaz dans le désaérateur 18 devient telle que l'eau atteint le niveau minimal dans l'enceinte 18a : à réception du signal du détecteur de niveau minimal, l'automate de l'installation commande l'arrêt de la pompe 17, la fermeture de la vanne d'arrêt 20 et la mise en marche de la pompe 22 pour assurer le remplissage en eau du désaérateur 18 et l'évacuation des gaz hors du circuit C. Le flux d'échantillon dans le circuit C peut ensuite être rétabli, par arrêt de la pompe 22, ouverture de la vanne 20 et remise en marche de la pompe 17. La pompe 22 peut également être utilisée, en procédant comme on vient de le décrire, pour remédier à des colmatages dans le circuit C, lesquels provoquent des diminutions de débit qui vont permettre de les détecter par l'intermédiaire du débitmètre 23.

De par sa conception, l'installation selon l'invention est auto-nettoyante en ce qu'il est possible de faire circuler dans le circuit C et l'ensemble du circuit de prélèvement, de l'eau chargée de chlore dans le chlorateur il à la base de la colonne 10. Pour le nettoyage du système de prélèvement, la conduite de refoulement est raccordée tour à tour avec chacune des trois conduites de prélèvement 1 à 3. S'agissant du circuit C, seule une petite partie regroupant la branche 11b du chlorateur, la conduite d'admission d'échantillon 16 et ses ramifications 16a et 16b ne pourra pas être soumise à un flux d'eau chlorée. En conséquence, il sera avantageux de prévoir que les parois de cette partie de circuit au contact du liquide soient constituées ou revêtues d'un matériau antifouling (antisalissures) tel que du cupronickel.

Toutes les opérations qui viennent d'être décrites (fonctionnement normal avec production d'informations, nettoyage des capteurs, nettoyage de l'ensemble de l'installation, ...) sont regroupées sous forme de cycle, programmé dans l'automate de commande en fonction des particularités du site d'implantation, de telle sorte que le besoin d'assistance humaine est réduit au minimum.

## Revendications

1. Installation de surveillance de la qualité d'un milieu liquide comprenant, associé à un système de prélèvement d'échantillon dans le milieu liquide, un circuit hydraulique (C) doté de capteurs de mesure et/ou de détection (12 à 15) dans lequel passe ledit échantillon, **caractérisée en ce que** ledit circuit (C) comprend d'amont en aval à partir de l'arrivée d'échantillon en provenance du système de prélèvement :
une colonne montante (10) le long de laquelle sont implantés lesdits capteurs (12 à 15) ;
une première pompe (17) au-dessus de la colonne montante assurant la circulation de l'échantillon dans ledit circuit (C) ;
un désaérateur (18) pour piéger les gaz transportés sous forme de bulles par l'échantillon liquide, lequel désaérateur (18) est placé au-dessus de ladite première pompe (17) ;
une conduite de refoulement (19) raccordée à la base dudit désaérateur (18) et pourvue d'une vanne d'arrêt (20) ;
une seconde pompe auto-amorçante (22) montée sur une conduite (21) raccordée au sommet dudit désaérateur (18), et
un moyen pour soumettre périodiquement les têtes d'au moins certains desdits capteurs à des jets de liquide sous pression pour en détacher les salissures et bulles gazeuses qui s'y fixent lors du passage de l'échantillon.

2. Installation selon la revendication 1, **caractérisé en ce qu'**un moyen générateur de chlore (11) est monté à la base de ladite colonne montante (10).

3. Installation selon la revendication 2, **caractérisée en ce que** ledit moyen générateur de chlore (11) est conçu de manière à former un fond de décantation pour ladite colonne montante (10).

4. Installation selon l'une des revendications 1 à 3, **caractérisée en ce que** dans au moins certaines parties du circuit (C) et du système de prélèvement, les éléments ou parois au contact de l'échantillon liquide sont constitués ou revêtus d'un matériau antifouling.

5. Installation selon la revendication 4, **caractérisée en ce que** ledit matériau antifouling est du cupronickel.

6. Installation selon l'une des revendications 1 à 5, **caractérisée en ce que** le désaérateur (18) comporte une enceinte cylindrique verticale (18a) communiquant par sa base avec la sortie de ladite première pompe (17) et la conduite de refoulement (19), et par son sommet avec l'entrée de ladite seconde pompe auto-amorçante (22), et **en ce qu'**il est pourvu d'un détecteur de niveau minimum de liquide dans l'enceinte (18a) pour lequel il convient d'évacuer les gaz recueillis.

7. Installation selon l'une des revendications 1 à 6, **caractérisée en ce que** ledit moyen pour soumettre des têtes de capteur à des jets de liquide sous pression comporte une conduite (24) dotée d'une troisième pompe (25), connectée en amont de celle-ci à la conduite de refoulement (19), et desservant en aval une pluralité de conduites secondaires (24a, 24b, 24c) se terminant chacune par un ajutage dirigé sur une tête de capteur.

8. Installation selon l'une des revendications 1 à 7, **caractérisée en ce qu'**un débitmètre (23) est prévu dans ledit circuit (C), destiné à régler le flux d'échantillon liquide dans le circuit (C), et à détecter des anomalies affectant ledit flux, telles que des colmatages.

9. Installation selon l'une des revendications 1 à 8, **caractérisée en ce que** ledit système de prélèvement d'échantillons comporte une pluralité de conduites de prélèvement (1 à 3) dont les orifice de prélèvement sont à des profondeurs différentes, lesquelles conduites (1 à 3) sont toutes raccordables sélectivement avec l'entrée et la sortie dudit circuit (C).

10. Installation selon l'une des revendications 1 à 9, **caractérisée en ce que** toute la partie dudit circuit (C) à partir de l'orifice de prélèvement d'échantillon jusqu'au désaérateur (18) est montante.

## Claims

1. Plant for monitoring the quality of a liquid medium comprising, associated with a system for taking a sample from the liquid medium, a hydraulic circuit (C) provided with measurement and/or detection sensors (12 to 15) in which the said sample passes, ***characterised by** the fact that* the said circuit (C) comprises from upstream to downstream from the arrival of the sample from the sampling system:
a rising column (10) along which the said sensors (12 to 15) are embedded;
a first pump (17) above the rising column for circulation of the sample in the said circuit (C);
a deaerator (18) to trap the gases conveyed in the form of bubbles by the liquid sample, which deaerator (18) is placed above the said first pump (17);
a delivery conduit (19) connected to the base of the said deaerator (18) and provided with a stop-valve (20);
a self-starting second pump (22) mounted in a pipe (21) connected to the top of the said deaerator (18), and
a means for periodically subjecting the heads of at least certain of the said sensors to jets of pressurised liquid to remove from them dirt and gas bubbles which become affixed to them on passage of the sample.

2. Plant as described in claim 1, ***characterised by** the fact that* a means for generating chlorine (11) is mounted at the base of the said rising column (10).

3. Plant as described in claim 2, ***characterised by** the fact that* the said means for generating chlorine (11) is so designed as to form a decanting bottom for the said rising column (10).

4. Plant as described in one of claims 1 to 3, ***characterised by** the fact that* in at least certain parts of the circuit (C) and of the sampling system, the elements or walls in contact with the liquid sample are formed of or coated with an anti-fouling material.

5. Plant as described in claim 4, ***characterised by** the fact that* the said anti-fouling material is cupro-nickel.

6. Plant as described in one of claims 1 to 5, ***characterised by** the fact that* the deaerator (18) includes a vertical cylindrical chamber (18a) communicating through its base with the output of the said first pump (17) and the delivery pipe (19), and through its top with the input of the said self-starting second pump (22), and by the fact that it is provided with a minimum liquid level detector in the chamber (18a) for which the collected gases have to be evacuated.

7. Plant as described in one of claims 1 to 6, ***characterised by** the fact that* the said means for subjecting sensor heads to jets of pressurised liquid include a pipe (24) provided with a third pump (25), connected upstream of this to the delivery conduit (19), and downstream serving a plurality of secondary pipes (24a, 24b, 24c) each terminated by a nozzle directed onto a sensor head.

8. Plant as described in one of claims 1 to 7, ***characterised by** the fact that* a flow-rate meter (23) is provided in the said circuit (C), intended to regulate the flow of liquid sample in the circuit (C), and to detect anomalies affecting the said flow, such as blockages.

9. Plant as described in one of claims 1 to 8, ***characterised by** the fact that* the said system for taking samples includes a plurality of sampling pipes (1 to 3) the sampling orifices of which are at different depths, which pipes (1 to 3) are all selectively connectable to the input and the output of the said circuit (C).

10. Plant as described in one of claims 1 to 9,
***characterised by** the fact that* the whole part of the said circuit (C) from the sampling orifice to the deaerator (18) is rising.

## Patentansprüche

1. Anlage zur Überwachung der Qualität eines flüssigen Milieus, die einem System zur Probenentnahme aus dem flüssigen Milieu zugeordnet einen mit Mess- und/oder Erfassungsfühlern (12 bis 15) versehenen Hydraulikkreis (C) aufweist, den die Probe durchquert, **dadurch gekennzeichnet, dass** der Kreis (C) von stromaufwärts nach stromabwärts ausgehend von der Ankunft der vom Entnahmesystem kommenden Probe aufweist:
ein Steigrohr (10), entlang dessen die Messfühler (12 bis 15) angeordnet sind;
eine erste Pumpe (17) oberhalb des Steigrohrs, die die Zirkulation der Probe im Kreis (C) gewährleistet;
eine Entlüftungseinrichtung (18), um die von der flüssigen Probe in Form von Blasen transportierten Gase einzufangen, wobei die Entlüftungseinrichtung (18) oberhalb der ersten Pumpe (17) angeordnet
ist;
eine Förderleitung (19), die an die Basis der Entlüftungseinrichtung (18) angeschlossen und mit einem Absperrschieber (20) versehen ist; eine zweite, selbstansaugende Pumpe (22), die auf eine Leitung (21) montiert ist, die mit der Oberseite der Entlüftungseinrichtung (18) verbunden ist, und
eine Einrichtung, um die Köpfe von mindestens einigen der Messfühler periodisch Druckflüssigkeitsstrahlen auszusetzen, um die Verschmutzungen und Gasbläschen davon zu lösen, die sich beim Durchgang der Probe dort festsetzen.

2. Anlage nach Anspruch 1, **dadurch gekennzeichnet, dass** eine Chlorerzeugungseinrichtung (11) an der Basis des Steigrohrs (10) angebracht ist.

3. Anlage nach Anspruch 2, **dadurch gekennzeichnet, dass** die Chlorerzeugungseinrichtung (11) so gestaltet ist, dass sie einen Klärboden für das Steigrohr (10) bildet.

4. Anlage nach einem der Ansprüche 1 bis 3, **dadurch gekennzeichnet, dass** zumindest in manchen Bereichen des Kreises (C) und des Entnahmesystems die Elemente oder Wände, die mit der flüssigen Probe in Kontakt stehen, aus einem Fäulnisschutzmaterial bestehen oder damit verkleidet sind.

5. Anlage nach Anspruch 4, **dadurch gekennzeichnet, dass** das Fäulnisschutzmaterial Kupfemickel ist.

6. Anlage nach einem der Ansprüche 1 bis 5, **dadurch gekennzeichnet, dass** die Entlüftungseinrichtung (18) einen senkrechten zylindrischen Raum (18a) aufweist, der an seiner Basis mit dem Ausgang der ersten Pumpe (17) und der Förderleitung (19) und an seiner Oberseite mit dem Eingang der zweiten, selbstansaugenden Pumpe (22) verbunden ist, und dass sie mit einem Detektor für den Mindestflüssigkeitspegel im Raum (18a) versehen ist, bei dem es angebracht ist, die gesammelten Gase zu evakuieren.

7. Anlage nach einem der Ansprüche 1 bis 6, **dadurch gekennzeichnet, dass** die Einrichtung, um die Messfühlerköpfe Druckflüssigkeitsstrahlen auszusetzen, eine mit einer dritten Pumpe (25) versehene Leitung (24) aufweist, die stromaufwärts vor der Pumpe mit der Förderleitung (19) verbunden ist und stromabwärts mehrere Sekundärleitungen (24a, 24b, 24c) versorgt, die je in einem Ansatzrohr enden, das auf einen Messfühlerkopf gerichtet ist.

8. Anlage nach einem der Ansprüche 1 bis 7, **dadurch gekennzeichnet, dass** ein Durchflussmesser (22) in dem Kreis (C) vorgesehen ist, der den Fluss an flüssiger Probe im Kreis (C) regulieren und den Fluss beeinträchtigende Anomalien wie zum Beispiel Verstopfungen feststellen soll.

9. Anlage nach einem der Ansprüche 1 bis 8, **dadurch gekennzeichnet, dass** das System zur Probenentnahme mehrere Entnahmeleitungen (1 bis 3) aufweist, deren Entnahmeöffnungen sich auf verschiedenen Tiefen befinden, wobei die Leitungen (1 bis 3) alle selektiv mit dem Eingang und dem Ausgang des Kreises (C) verbunden werden können.

10. Anlage nach einem der Ansprüche 1 bis 9, **dadurch gekennzeichnet, dass** der ganze Bereich des Kreises (C) ausgehend von der Probenentnahmeöffnung bis zur Entlüftungsvorrichtung (18) ansteigt.
